(19) Europäisches Patentamt
European Patent Office
Office européen des brevets

(11) Publication number: **0 170 211 A1**

(12) **EUROPEAN PATENT APPLICATION**

(21) Application number: 85109349.2

(22) Date of filing: 25.07.85

(51) Int. Cl.$^4$: **C 07 C 101/28**
C 07 C 99/00, C 07 B 53/00

(30) Priority: 03.08.84 IT 2222684

(43) Date of publication of application: 05.02.86 Bulletin 86/6

(84) Designated Contracting States: AT BE CH DE FR GB LI LU NL SE

(71) Applicant: SIRAC S.p.A.
Via Calabiana 18
Milan(IT)

(72) Inventor: Miletti, Sandro
Via Casamorata 32
I-50139 Florence(IT)

(72) Inventor: Carobbi, Renato
via Dalmazia 168
Pistoia(IT)

(74) Representative: Gervasi, Gemma et al,
Studio Brevetti e Marchi NOTARBARTOLO & GERVASI
33, Viale Bianca Maria
I-20122 Milano(IT)

(54) **Process for preparing L-phenylalaninemethylester hydrochloride.**

(57) A new process for preparing L-phenylalaninemethylester hydrochloride (I):

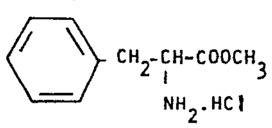

(I)

based on the stereospecific reduction of the compound

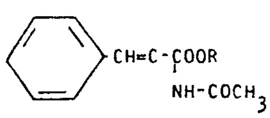

(IV)

in which R is H or $C_1$-$C_4$ alkyl, in the presence of a catalyst constituted by $(RhC1X_n)_2$, in which X is olefin or diolefin and n is 1 or 2, and by (+)DIOP in which (+)DIOP is (+)-2,3-0-isopropylidene-2,3-dihydroxy-1,4-bis-(diphenylphosphino)butane.

The procedure is conducted with a molar ratio of substrate (IV) to Rh of between 15,000/1 and 100.000/1, to obtain a practically 100% reduction yield and a product (I) of optical purity, ie 98-99%.

EP 0 170 211 A1

# PROCESS FOR PREPARING L-PHENYLALANINEMETHYLESTER HYDROCHLORIDE

This invention relates to a new process for preparing L-phenylalanine-methylester hydrochloride (I):

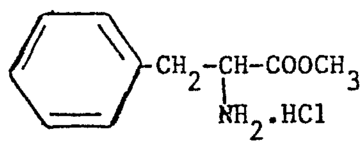

(I)

More particularly, the invention relates to a new process for preparing L-phenylalaninemethylester hydrochloride (I) based on the stereospecific reduction of α-acetamidocinnamic acid, or of an α-acetamidocinnamic acid ester, which lie within the formula:

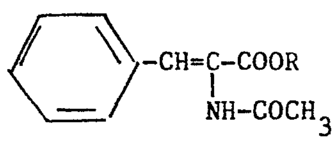

(IV)

in which R is H or $C_1$-$C_4$ alkyl.

The L antipode of N-acetylphenylalanine has lately assumed an increasing importance in that it constitutes a component of dipeptide-based sweeteners.

Various methods are known for preparing this compound. The most widely used method consists of the classical resolution of the raceme amino acid into its relative optical antipodes by converting a suitable derivative and crystallising the diastereoisomer salts obtained.

However, with this method it is not possible in the best of cases to

obtain more than 50% of the required enantiomer.

Methods are also known based on the stereospecific reduction of α-acetamidocinnamic acid catalysed by a chiral diphosphine and a complex of rhodium with an alkene or an alkadiene.

These methods are described for example in Czechoslovakian patent 211584 and in J. Am. Chem. Soc. (94:18) Sept. 6, 1972, pages 6429-6433.

However, these methods have not found industrial application in that the rhodium quantity to be used is very large, and the recovery of the rhodium, which is a very costly metal, is very expensive. In the best case (Czechoslovakian patent 211584) the molar ratio of the α-acetamidocinnamic acid to be reduced with respect to the rhodium is 1,350/1.

We have now discovered a new process for preparing L-phenylalanine by the stereospecific reduction of α-acetamidocinnamic acid or one of its esters, in which the implementation of certain critical operational steps has surprisingly enabled very high molar ratios of between 15,000/1 and 100,000/1 to be obtained between the substance to be reduced and the rhodium.

Moreover, said process allows a reduction yield close to 100% and an optical purity close to 100% to be obtained.

Because of the high cost of Rh catalysts and thus the high incidence of the catalyst cost on the process economy, the saving in catalyst and the simplification of the recovery operations, together with the high yield and high purity of the final product, make the new process industrially applicable in a very economical manner compared with known processes.

The process for preparing L-phenylalaninemethylester hydrochloride (I) by the stereospecific reduction of a compound of formula:

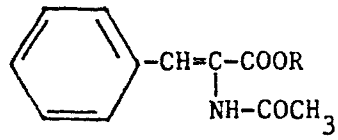 (IV)

in which R is H or $C_1$-$C_4$ alkyl, in the presence of a catalyst constituted by $[RhClX_n]_2$, in which X is olefin or diolefin and n is 1 if X is diolefin and 2 if X is olefin, and by (+)DIOP where (+)DIOP is (+)-2,3-O-isopropylidene-2,3-dihydroxy-1,4-bis(diphenylphosphino)-butane, according ot the present invention, is characterised in that the catalyst formation, by reaction between the rhodium complex and (+)DIOP, takes place in the presence of the substrate (IV) and in the absolute absence of air and/or oxygen, in a hydrogen or nitrogen environment. One of the preferred operational sequences is the following: the rhodium complex is dissolved in the alcoholic or aqueous-alcoholic solution of the substrate (IV), air is removed from the system and hydrogen is introduced while maintaining the temperature between 30° and 50°C, the (+)DIOP is added in the solid state in a hydrogen environment and dissolves to form the catalytic system with the rhodium complex in-situ, after which the compound (IV) is reduced stereospecifically by hydrogen at a pressure of between 0.1 and 100 atm.

Alternatively, the reaction between the rhodium complex and the (+)DIOP can take place in a nitrogen environment, before introducing the hydrogen.

The product of the reduction is then esterified and deacetylated if R is H, or is simply deacylated if R is $C_1$-$C_4$ alkyl, in the presence of gaseous HCl.

With this procedure, the product (I) is obtained if R is $HOCH_3$. If R is $C_2$-$C_4$ alkyl, a further transesterification stage is required.

These and further characteristics and advantages of the process according to the present invention will be more apparent from the detailed description given hereinafter.

As stated, the process according to the invention is applied either to the stereospecific reduction of ∝ -acetamidocinnamic acid or to the stereospecific reduction of its esters.

Obviously, as the final product of the process is L-phenylalaninemethylester hydrochloride (I), it is decidedly advantageous to use the methyl ester as the starting substance rather than the acid or esters other than the methyl.

The starting compounds (IV) are obtainable from azlactone (V), depending on the treatment conditions, in accordance with the following scheme:

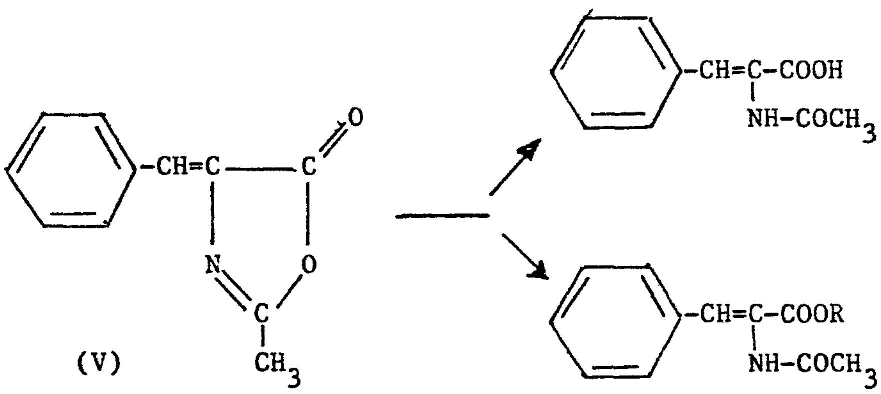

(V) (II) (III)

in which R is as heretofore defined.

More precisely, if the azlactone (V) is treated in an aqueous or, preferably, acetonic environment, the acid (II) is obtained, whereas if it is treated in an alcoholic medium in the presence of a strong base such as sodium hydroxide or sodium methylate, the ester (III) is obtained in which R varies according to the alcohol used.

The product to be reduced (II) or (III) is fed into the reactor in which the reduction is to be carried out, followed by the alcohol or water-alcohol in a quantity such as to obtain a solution of concentration between 100 g/l and 300 g/l, and preferably 200-250 g/l.

The rhodium complex is added in such a quantity as to obtain a molar ratio of the compound to be reduced with respect to the Rh of between 15,000/1 and 100,000/1.

The preferred rhodium complex is $[RhCl(COD)]_2$ where COD is 1,5-cyclooctadiene, but complexes with monoolefins can also be used, such as ethylene:$[RhCl(C_2H_4)_2]_2$, or cyclooctene:$[RhCl(C_8H_{14})_2]_2$.

The molar ratio of the DIOP to the $[RhClX_n]_2$ is between 2 and 0.1. The reduction of the product (IV) by hydrogen is conducted at a pressure of between 0.1 and 100 atm and at a temperature preferably of between 30° and 50°C.

After 1-2 hours the reduction is practically complete.

In reducing the product (IV) in which R is H, N-acetylphenylalanine

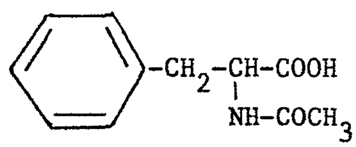

(VI)

is obtained, whereas in reducing the product (IV) in which R is alkyl, the N-acetylphenylalanine alkyl ester (VII)

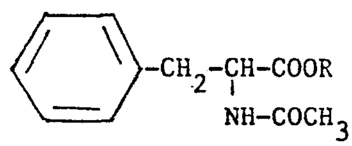

(VII)

is obtained.

In the case of the product (VI), the process continues with esterification and deacetylation.

These two reactions are implemented in a single stage by treatment with methanol (solvent used for the reduction) and gaseous hydrochloric acid, at a temperature of between 40° and 130°C.

In the case of the product (VII), if R is $CH_3$, only deacetylation is required, and this is again effected by treatment with methanol (solvent used for the reduction) and gaseous hydrochloric acid, at a temperature of between 40° and 130°C.

Thus in both cases, the same final product, namely L-phenylalaninemethylester hydrochloride (I), is obtained. However, the time required

for simple deacetylation is shorter than the time required for the two reactions, esterification and deacetylation.

As already stated, if R is other than H or $CH_3$, a final transesterification stage is still required after the deacylation.

This means that it is more convenient to use as the starting substance the methyl ester of α -acetamidocinnamic acid rather than α -acetamidocinnamic acid itself or its esters other than the methyl.

However, in all cases the yield of the reduction reaction is close to 100% and the optical purity of the product obtained is of the order of 98-99%.

The following examples of the process according to the present invention are described for illustrative but non-limitative purposes.

EXAMPLE 1

137.76 g (0.672 moles) of α -acetamidocinnamic acid are dissolved in 500 ml of methanol in a reactor fitted with an agitator, a system for applying vacuum, and a system for feeding nitrogen and hydrogen.

9.59 mg (0.0388 mmoles) of Rh in the form of $[RhCl(COD)]_2$ in the solid state are then added under agitation.

Air is removed from the resultant solution and from the apparatus, firstly by applying vacuum and then by introducing nitrogen.

The procedure is repeated three times and hydrogen is then introduced.

The solution is heated to 37°C and 19.39 mg (0.0388 mmoles) of (+)DIOP in the solid state are added in a hydrogen atmosphere and out of contact with air.

Hydrogen is then fed into the reactor to a pressure of 1 atmosphere.

After about 1.5 hours, the reduction of the ∝ -acetamidocinnamic acid is complete, to obtain 137 g (0.66 moles) of N-acetylphenylalanine, with a reduction yield of 98.4%.

When hydrogenation is complete, the solution is passed through an ion exchange resin to recover the Rh.

In order to effect the deacetylation and methyl esterification, the N-acetylphenylalanine, in its methanol solution from which the rhodium has been removed, is treated with gaseous hydrochloric acid under reflux for a time of about 40 hours.

In this manner, L-phenylalaninemethylester hydrochloride (I) is obtained, and is separated from the reaction mixture by concentration. Optical purity 99%.

EXAMPLE 2

148.48 g (0.677 moles) of the methyl ester of ∝ -acetamidocinnamic acid in 500 ml of methanol were fed into the reactor of Example 1.

9.59 mg (0.0388 mmoles) of Rh in the form of $[RhCl(COD)]_2$ in the solid state were added under agitation.

The air was removed from the resultant solution and from the apparatus by successive repeated applications of vacuum and nitrogen introduction.

Hydrogen was then fed into the reactor.

The solution was heated to 40°C and 19.39 mg (0.0388 mmoles) of (+)DIOP in the solid state were added in a hydrogen atmosphere.

Hydrogen was then fed to a pressure of 1 atmosphere. After about 1 hour, the ester reduction was complete, and 147 g (0.665 moles) of N-acetylphenylalaninemethylester were obtained with a reduction yield of 98%.

On completion of hydrogenation, the solution was passed through an ion exchange resin for recovery of the rhodium.

The deacetylation is effected by treating the methanolic solution with gaseous HCl under reflux for 30 hours.

The L-phenylalaninemethylester hydrochloride (I) is separated from the reaction mixture by concentration, and has an optical purity of 99%.

EXAMPLE 3

Example 1 is repeated by feeding the came quantity of $[RhCl(COD]_2$, methanol and α-acetamidocinnamic acid into the reactor. Air is removed from the resultant solution and apparatus, firstly by applying vacuum and then by introducing nitrogen. At this point, 19.39 mg (0.0388 mmoles) of (+)DIOP in the solid state are introduced, in a nitrogen environment and without contact with air.

The mixture is heated to 40°C and hydrogen is introduced to a pressure of 1 atmosphere. On proceeding in the same manner as Example 1, analogous results are obtained.

EXAMPLE 4

The same quantities of methanol and α-acetamidocinnamic acid are fed into the reactor of Example 1.

9.59 mg of $[RhCl(COD)]_2$ and 19.39 mg of (+)DIOP in the solid state are placed in a cup so as not to be in contact with the solution. Air is removed from the solution and from the system by carrying out the previously described vacuum-nitrogen procedure three times.

Finally, the nitrogen is also removed and is replaced by hydrogen. At this point, the rhodium complex and the (+)DIOP are allowed to fall into the solution, and hydrogenation is effected under the same conditions as Example 1, to obtain analogous results.

EXAMPLE 5

9.59 mg of $[RhCl(COD)]_2$ are dissolved in 100 cc of methanol. The solution is introduced into a small bomb. Air is then removed from the system by 3 successive repeated applications of vacuum and nitrogen introduction.

The nitrogen is then also removed and replaced by hydrogen at a pressure of 5 atmospheres.

A solution of 19.39 mg of (+)DIOP in 100 cc of methanol is fed into another small bomb.

Air is removed from the system by 3 successive repeated applications of vacuum and nitrogen introduction.

The nitrogen is then removed and replaced by hydrogen at a pressure of 5 atmospheres.

148.48 g of the methyl ester of $\alpha$-acetamidocinnamic acid and 300 cc of methanol are fed into a reactor fitted with an agitator, a system for applying vacuum and for feeding nitrogen and hydrogen, and two valves fitted to two connectors for connection to the bombs. Air is removed by successive repeated applications of vacuum and nitrogen introduction. Vacuum is then applied and hydrogen fed to a pressure of 1 atmosphere. At this point, the reactor is connected to the two bombs containing the rhodium complex and (+)DIOP solutions, which are fed into the reactor under pressure. Hydrogenation is effected in the same manner as Example 2, to obtain analogous results.

PATENT CLAIMS

1. A process for preparing L-phenylalaninemethylester hydrochloride (I)

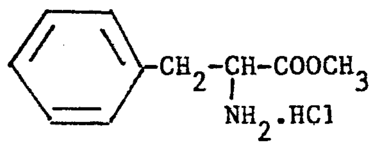

(I)

by stereospecifically reducing with hydrogen a compound of formula

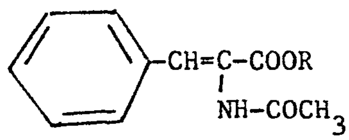

(IV)

in which R is H or $C_1$-$C_4$ alkyl, in the presence of a catalyst constituted by $[RhClX_n]_2$, in which X is olefin or diolefin and n is 1 if X is diolefin and is 2 if X is monoolefin, and by (+)DIOP in which (+)DIOP is (+)-2,3-O-isopropylidene-2,3-dihydroxy-1,4-bis(diphenylphosphino)butane, characterised in that the catalyst is prepared by reacting $[RhClX_n]_2$ and (+)DIOP in the solution of the substrate (IV) in the absolute absence of air and/or oxygen, in a nitrogen or hydrogen environment, the product of the reduction being subsequently esterified and/or deacylated and possibly transesterified with methy alcohol.

2. A process as claimed in claim 1, wherein the rhodium complex is $[RhCl(COD)]_2$, COD being 1,5-cyclooctadiene.

3. A process as claimed in claim 1, wherein (+)DIOP is added in the solid state in a nitrogen environment to an alcoholic or aqueous-alcoholic solution of substrate (IV) and $[RhClX_n]_2$, which has been previously deaerated by successive vacuum and nitrogen-introduction treatments.

4. A process as claimed in claim 3, wherein DIOP is added to the system in a hydrogen environment.

5. A process as claimed in claim 1, wherein $[RhClX_n]_2$ and (+)DIOP are both added in the solid state to an aqueous or aqueous-alcoholic

solution of substrate (IV), which has been previously deaerated by successive vacuum and nitrogen-introduction treatments.

6. A process as claimed in claim 5, wherein $[RhClX_n]_2$ and (+)DIOP are added to the system in a hydrogen environment.

7. A process as claimed in claim 1, wherein said solution of the substrate (IV) is an alcoholic or aqueous-alcoholic solution of concentration between 100 and 300 g/l.

8. A process as claimed in claim 1, wherein the catalyst is prepared at a temperature of between 30° and 50°C.

9. A process as claimed in claim 1, wherein the hydrogenation is effected at a temperature of between 30° and 50°C.

10. A process as claimed in claim 1, wherein said stereospecific reduction with hydrogen is effected at a pressure of between 0.1 and 100 atm.

11. A process as claimed in claim 1, wherein the molar ratio of the substrate (IV) to the rhodium is between 15,000/1 and 100,000/1.

12. A process as claimed in claim 1, wherein the molar ratio of DIOP to Rh is approximately 1:1.

13. A process as claimed in claim 1, wherein said esterification and/or deacetylation of the product of the reduction are conducted in a single stage, in a methanol environment in the presence of gaseous HCl, under reflux.

14. L-phenylalaninemethylester hydrochloride (I) of optical purity equal to 98-99%, when obtained by the process claimed in claims 1 to 13.

European Patent Office

# EUROPEAN SEARCH REPORT

0170211

Application number: EP 85109349.2

## DOCUMENTS CONSIDERED TO BE RELEVANT

| Category | Citation of document with indication, where appropriate, of relevant passages | Relevant to claim | CLASSIFICATION OF THE APPLICATION (Int Cl 4) |
|---|---|---|---|
| D,X | JOURNAL OF THE AMERICAN CHEMICAL SOCIETY, vol. 94, August-October 1972, H.B. KAGAN et al. "Asymmetric Catalytic Reduction with Transition Metal Complexes" * Pages 6429-6433 --- | 1,4,12 14 | C 07 C 101/28<br>C 07 C 99/00<br>C 07 B 53/00 |
| X | GMELIN HANDBOOK OF INORGANIC CHEMISTRY, edition 8, Rh Rhodium Suppl. vol. B3, 1984 SPRINGER VERLAG, Berlin, Heidelberg, New York, Tokyo pages 185-187 * Page 185, lines 21-23; page 186, lines 21-23 * ---- | 1 | |
| | | | **TECHNICAL FIELDS SEARCHED (Int Cl 4)** |
| | | | C 07 C 101/00<br>C 07 B |

The present search report has been drawn up for all claims

| Place of search | Date of completion of the search | Examiner |
|---|---|---|
| VIENNA | 15-10-1985 | HOFBAUER |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1503 03 82